# EUROPEAN PATENT APPLICATION

(11) **EP 4 822 688 A1**
(43) Date of publication of application: **14.10.2026**
(21) Application number: 25170128.0
(22) Date of filing: 11.04.2025
(51) Int. Cl.: G10L 15/26

(54) **SYSTEM, METHOD, AND COMPUTER PROGRAM FOR A SURGICAL OR SCIENTIFIC IMAGING SYSTEM, SURGICAL OR SCIENTIFIC IMAGING SYSTEM**

(71) Applicant: Leica Instruments (Singapore) Pte Ltd., Singapore 618299 (SG)
(72) Inventor: THEMELIS, George, 618299 Singapore (SG)
(74) Representative: 2SPL Patentanwälte PartG mbB

(57) **Abstract**

Examples relate to a system (110), a method and a computer program for a surgical or scientific imaging system (100), and to a surgical or scientific imaging system comprising such a system. The system (110) is configured to obtain a natural language input of a user of the surgical or scientific imaging system, process the natural language input using a large language model, wherein the large language model is trained to output, based on the natural language input of the user, at least one parameter for controlling an operation of the surgical or scientific imaging system, and control the surgical or scientific imaging system based on the output of the large language model.

## Description

### Technical field

Examples relate to a system, a method, and a computer program for a surgical or scientific imaging system, and to a surgical or scientific imaging system comprising such a system.

### Background

High-end digital microscopes, such as those used as part of surgical microscope systems, offer a plethora of functionality through the use of digital image processing working hand in hand with digital control of the optical components of the microscope. While this makes such microscopes extremely powerful and useful, the sheer number of possibilities makes it harder for users to utilize the full potential of their microscope.

There may be a desire for an improved concept for operating a surgical or scientific imaging device, such as a microscope, which makes it easier for users to utilize the full potential of their surgical or scientific imaging device.

### Summary

This desire is addressed by the subject matter of the independent claims.

Various examples of the present disclosure are based on the finding, that large language models (LLMs) are highly capable of processing large amounts of structured or unstructured information to establish a context that is used to interpret a request by a user. This context can be given to the LLM during training or fine-tuning or can be provided to the LLM as part of a prompt. The context can then be used to interpret the natural language of users attempting to achieve a result while using a surgical or scientific imaging device, such as a surgical microscope. In previous iterations, natural language control of microscopes has been limited to specific keywords, such as "autofocus", "zoom in" or "zoom out". The proposed LLM-based processing of natural language provided by a user uses the context to determine the functionality required to achieve the desired outcome specified by the user, without the user having to specify which functionality is to be used for this purpose. This allows the user to use the potential of the respective surgical or scientific imaging device more fully.

Some aspects of the present disclosure relate to a system for a surgical or scientific imaging system. The system comprises one or more processors and one or more storage devices. The system is configured to obtain a natural language input of a user of the surgical or scientific imaging system. The system is configured to process the natural language input using a large language model. The large language model is trained to output, based on the natural language input of the user, at least one parameter for controlling an operation of the surgical or scientific imaging system. The system is configured to control the surgical or scientific imaging system based on the output of the large language model. By processing the natural language input of the user using an LLM, learned or provided context regarding the functionality of the surgical or scientific imaging system can be used to interpret the natural language input. This way, the LLM can determine the functionality required to achieve the desired outcome specified by the user without the user having to specify which functionality is to be used for this purpose. This allows the user to use the potential of the respective surgical or scientific imaging device more fully.

In many cases, the user may use natural language to control the surgical or scientific imaging system while actively using it, e.g., during a surgical procedure. In these cases, the user often wishes to achieve a result that is related to what they are currently perceiving through the surgical or scientific imaging system. To improve the LLM's understanding of the natural language input, image data of what the user is currently seeing may be provided to the LLM as further input. In other words, the system may be configured to obtain image data of an imaging device of the surgical or scientific imaging system. The system may be configured to provide the image data or data derived from the image data as further input to the large language model. For example, the large language model may be trained to output, based on the natural language input of the user, and based on the image data or data derived from the image data, the at least one parameter for controlling the operation of the surgical or scientific imaging system. This way, the large language model is enabled to derive the at least one parameter also based on what the user is seeing.

In surgical contexts, the visualization provided by the surgical imaging device often relates to specific features in the images, such as anatomical features (tissue, vessels, etc.), surgical tools or specific pointers (e.g., fiducial pointers or the user pointing at something, e.g., using a scalpel). This valuable information allows the LLM to better understand the natural language input by the user. Accordingly, the image data may be image data of a surgical site. In this case, the system may be configured to pre-process the image data to determine at least one of one or more anatomical features, one or more surgical tools or one or more pointers shown in the image data. The system may be configured to provide information on at least one of the one or more anatomical features, the one or more surgical tools or the one or more pointers as the data derived from the image data as further input to the large language model. This enables the large language model to derive the at least one parameter also based on specific features in the image representing what the user is seeing.

As an alternative to preprocessing the image data, processing of the image data may be performed by the LLM. Accordingly, with the image data being image data of a surgical site, the large language model may be trained to determine at least one of one or more anatomical features, one or more surgical tools or one or more pointers shown in the image data. The large language model may be trained to provide the at least one parameter for controlling the operation of the surgical or scientific imaging system based on at least one of the one or more anatomical features, the one or more surgical tools or the one or more pointers. This avoids the computationally expensive preprocessing of the image data while transferring the task to the LLM (which may be hosted by a more powerful computer outside the surgical or scientific imaging system).

In many cases, not all portions of the image shown in the image data are equally relevant. In many cases, the user is focusing on a specific portion of the image, which likely relates to the users' natural language input. For example, the system may be configured to obtain information on a gaze of a user of the surgical or scientific imaging system, and to provide information on the gaze as further input to the large language model. This way, the LLM can provide the at least one parameter based on the specific portion of the image that the user is actively looking at.

While the aforementioned additional inputs provide valuable context for the LLM, they also increase the number of input tokens being processed by the LLM, and thus the time it takes to provide an output. As the additional inputs are not always necessary or useful, in many cases, they can be omitted without deteriorating the output, while decreasing the time it takes to provide the output. This can be achieved by prompting the LLM to use function calling to obtain the additional input(s) as needed, e.g., based on the natural language input. Accordingly, the large language model may be trained to retrieve at least one of image data, data derived from the image data, or information on a gaze of a user of the surgical or scientific imaging system using a function calling functionality.

In some cases, it may be challenging to achieve the result desired by the user in one go. In particular, the interaction of different functionalities of the surgical or scientific imaging device may lead to outcomes that are within an expected range but not precisely the desired result. In such cases, the LLM may work iteratively, changing the at least one parameter over time as it approaches the desired result based on information on the state of the surgical or scientific imaging system. This information on the state of the surgical or scientific imaging system allows the LLM to determine how close the result is to the desired result. Accordingly, the system may be configured to provide, after controlling the surgical or scientific imaging system based on the output of the large language model, information on a state of the surgical or scientific imaging system as input for the large language model. The large language model may be trained to output an update to the at least one parameter for controlling the operation of the surgical or scientific imaging system based on the information on the state. This way, the handling of complex user queries can be improved.

In some cases, the LLM can not only be used to change parameters of the surgical or scientific imaging system but also for detecting events such as bleeding. Accordingly, the system may be configured to provide at least one of information on a state of the surgical or scientific imaging system, image data of an imaging device of the surgical or scientific imaging system, or data derived from the image data of the imaging device of the surgical or scientific imaging system as input for the large language model. The large language model may be trained to determine one or more events to be detected based on the natural language input of the user, detect the one or more events in the information on the state of the surgical or scientific imaging system, the image data or the data derived from the image data, and provide an output indicating the one or more events having occurred. This way, the LLM acts as second pair of eyes for the user, making the user aware of relevant events while using the surgical or scientific imaging system.

The at least one parameter may be used to control various aspects of the surgical or scientific imaging system. For example, basic functionality, such as repositioning, zooming or focusing may be performed based on the user's natural language input. In other words, the system may be configured to control the position (and orientation) of an imaging device of the surgical or scientific imaging system relative to a sample being observed using the imaging device based on the at least one parameter. Additionally, or alternatively, the system may be configured to control at least one of a zoom functionality or a focus functionality of an imaging device of the surgical or scientific imaging system based on the at least one parameter. These functionalities are often required during the use of the surgical or scientific imaging system. They benefit from the additional context understanding provided by the LLM, as the LLM enables repositioning, zooming, or focusing based on specific features shown in the image data and described by the user.

Another area where the LLM can be useful is in digital image processing. Surgical or scientific imaging devices often have a number of different imaging modalities, i.e., different ways of capturing images of a sample. In addition, they have digital image processing operations that can be applied to the image data provided by the imaging modality(s), resulting in a near-infinite number of possibilities to generate and process images. By training or providing the LLM with information on what is best captured using which imaging modalities, and information on the effects of different image processing operations, the LLM can be used to generate or re-configure an image processing pipeline to achieve a desired visual result based on the user's natural language input. Accordingly, the large language model may be trained to output information on a configuration of an image processing pipeline based on the natural language input of the user. The system may be configured to process image data or pre-processed image data of an imaging device of the surgical or scientific imaging system using an image processing pipeline that is based on the information on the configuration of the image processing pipeline output by the large language model. This way, an image processing pipeline may be created or refined based on a desired visualization described by the user.

In general, an image processing pipeline starts with image data provided by at least one of the imaging modalities (e.g., white light imaging, various fluorescence imaging modalities) of the imaging device. This image data is then processed using image processing operations provided by the surgical or scientific imaging processing system. The LLM can create or modify an image processing pipeline by selecting one or more image processing operations (and the order in which they are used) that are to be included in the image processing pipeline. In other words, an image processing pipeline may comprise a sequence of image processing operations. The large language model may be trained to select one or more image processing operations for the image processing pipeline from a library of available image processing operations based on the natural language input of the user. This way, an image processing pipeline may be created or refined based on a desired visualization described by the user.

For example, the library of available image processing operations may comprise at least one of one or more mathematical operations, one or more morphological operations, a gamma adjustment operation, one or more thresholding operations, a sharpness adjustment operation, one or more histogram-based operations, an image segmentation operation, or an object detection operation. These are operations that are commonly provided by surgical or scientific imaging systems.

As outlined above, the image processing pipeline starts with image data of one of the imaging modalities of the imaging device. In addition, or as an alternative, to selecting image processing operation(s), the LLM may be tasked with selecting suitable image data (e.g., raw image data or image data that is pre-processed into an intermediate representation). In other words, the image processing pipeline may be configured to process image data or pre-processed image data of the imaging device of the surgical or scientific imaging system. The large language model may be trained to select one or more instances of a plurality of available types of image data or pre-processed image data based on the natural language input of the user. This way, another degree of freedom may be provided to the LLM to determine a suitable image processing pipeline.

In general, the LLM may need to know the properties of the different image processing operations and/or of the types of image data. These properties can be provided to the LLM as textual information, e.g., as part of the prompt or system message. In other words, the system may be configured to provide a data structure comprising information on available image processing operations and/or information on available types of image data or pre-processed image data as input for the large language model. In this case, the LLM can easily be made aware of new image processing operations becoming available or new types of image data becoming available, or be provided with improved descriptions over time, at the cost of increasing the number of tokens to be processed by the LLM. Alternatively, or additionally, the LLM may be trained based on the respective information. In other words, the large language model may be trained based on the data structure comprising information on available image processing operations and/or information on available types of image data or pre-processed image data. This way, the information does not have to be provided at runtime, which decreases the token count, with the caveat that the LLM has to be retrained when new image processing operations or new types of image data become available.

The aim with respect to image processing pipelines is to enable the user to describe what he or she wants to see. For example, the user may provide the following natural language input: "Show me the anatomy in black and white, the ICG fluorescence in green, and the 5-ALA in red". If the LLM is trained on, or provided with, information on which type of image data is suitable for showing the anatomy (white light image data), the ICG fluorescence (a first type of fluorescence image data), and the 5-ALA (a second type of fluorescence image data), it can select the appropriate types of image data. Then it can select the operations for coloring the respective image data. For example, the white light image data can be converted into a black and white image (or rather a greyscale image), and the first type of fluorescence image data can be multiplied with green color, and the second type of fluorescence image data can be multiplied with red. Then, the image data can be overlaid over each other, with the black and white-colored white light image as a base layer. Thus, based on the description of what the user desires to see, the LLM can select suitable types of image data and corresponding image processing operations. In other words, the large language model may be trained to output the information on the configuration of the image processing pipeline based on natural language describing a desired visual output of the image processing pipeline.

The proposed generation or modification of image processing pipelines is designed to be used (also) while using the surgical or scientific imaging system, e.g., during surgery. To avoid situations where the user is provided with a sub-par view, some functional tests may be performed on the image processing pipeline before it is activated. In other words, the system may be configured to perform a pre-defined set of functional tests on the image processing pipeline before processing the image data or pre-processed image data of an imaging device of the surgical or scientific imaging system using the image processing pipeline. This way, situations can be avoided in which the user needs to interrupt their work switch to a different image processing pipeline because the generated or modified image processing pipeline is unusable.

The determination of various aspects of an image processing pipeline relates to the acquisition, processing, and output (displaying) of image data obtained via at least one optical imaging sensor of the imaging device. Accordingly, the at least one parameter may include a setting related to at least one of image acquisition, image processing, or display.

The proposed concept is not limited to using a microphone to input the natural language input as spoken voice to the system. As an alternative, other means of inputting natural language may be used, such as written text or sign language. Accordingly, the natural language input may include at least one of a voice command, a text input, and a sign language input.

Another aspect of the present disclosure relates to a surgical or scientific imaging system comprising the aforementioned system.

Another aspect of the present disclosure relates to a corresponding method for a surgical or scientific imaging system. The method comprises obtaining a natural language input of a user of the surgical or scientific imaging system. The method comprises processing the natural language input using a large language model. The large language model is trained to output, based on the natural language input of the user, at least one parameter for controlling an operation of the surgical or scientific imaging system. The method comprises controlling the surgical or scientific imaging system based on the output of the large language model.

Another aspect of the present disclosure relates to a computer program having a program code for performing the above method when the program is executed on processor.

### Short description of the Figures

Some examples of apparatuses and/or methods will be described in the following by way of example only, and with reference to the accompanying figures, in which:
- Fig. 1a: shows a schematic diagram of an example of a system for a surgical or scientific imaging system;
- Fig. 1b: shows a schematic diagram of a surgical imaging system;
- Fig. 2: shows an example of LLM-based control of a surgical imaging system;
- Fig. 3: shows an example of Al (Artificial Intelligence)-driven custom microscope imaging mode creation using natural language commands;
- Fig. 4: shows an example of a method for a surgical or scientific imaging system; and
- Fig. 5: shows an example of a system comprising an imaging device and a computer system.

### Detailed Description

Various examples will now be described more fully with reference to the accompanying drawings in which some examples are illustrated. In the figures, the thicknesses of lines, layers and/or regions may be exaggerated for clarity.

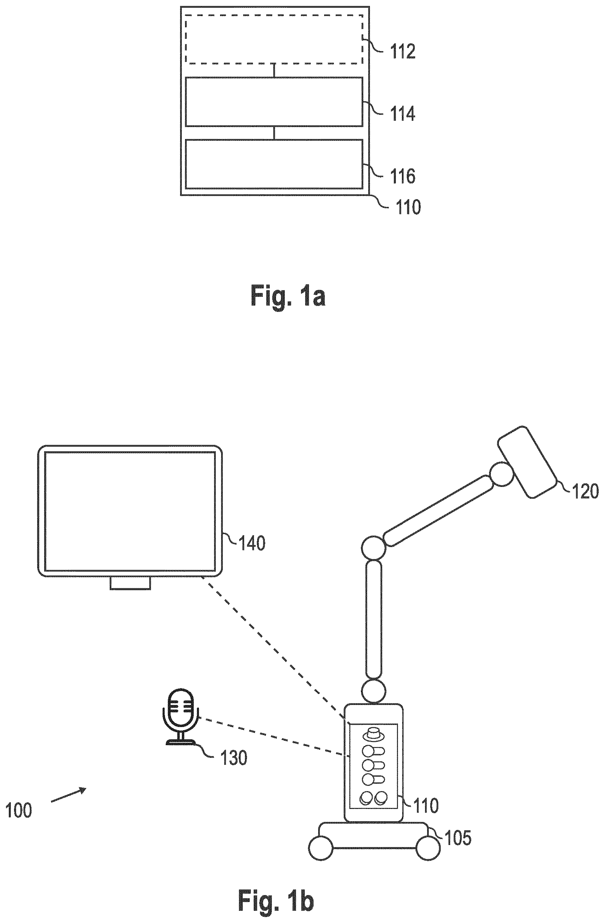

Fig. 1a shows a schematic diagram of an example of a system 110 for a surgical or scientific imaging system 100 (shown in Fig. 1b). The system 110 is a component of the surgical or scientific imaging system 100 and may be used to control various aspects of the surgical or scientific imaging system 100. In particular, it may be used for acquisition and/or processing of imaging data by at least one optical imaging sensor of an imaging device 120 of the surgical or scientific imaging system, through various means that will be introduced in more detail in the following. Additionally, the system 110 may be configured to control additional aspects of the surgical or scientific imaging system 100, e.g., to provide a display signal for one or more display devices 140 of the surgical or scientific imaging system.

In general, the system 110 may be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical or scientific imaging system 100 and outside the surgical or scientific imaging system 100, such as an optical imaging sensor of the imaging device 120 or a microphone 130), and with the one or more storage devices 116 (for storing information, such as machine-readable instructions of a computer program being executed by the one or more processors). In general, the functionality of the one or more processors 114 may be implemented by the one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical or scientific imaging system 100, which comprises various components in addition to the system 110. For example, the surgical or scientific imaging system 100 comprises the imaging device 120, and may comprise one or more additional components, such as one or more display devices 140. Fig. 1b shows a schematic diagram of an example of such a surgical or scientific imaging system 100, in particular, a surgical microscope system 100. In the following, the surgical or scientific imaging system 100 may also be referred to as the surgical microscope system 100. A surgical microscope system is a surgical imaging system 100 that comprises a (surgical) microscope as imaging device 120. However, the proposed concept is not limited to such embodiments. The surgical or scientific imaging system 100 may be based on various (single or multiple) imaging devices, such as one or more microscopes, one or more endoscopes, and/or one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars, exoscopes are only used via display modalities, such as a monitor or a head-mounted display. Accordingly, the surgical or scientific imaging system 100 may alternatively be a surgical endoscope system, or a surgical exoscope system. Yet alternatively, the surgical or scientific imaging system may be a scientific imaging system comprising a microscope, e.g., a laboratory microscope. However, the following illustrations assume that the surgical imaging device 120 is a surgical microscope, and that the surgical or scientific imaging system 100 is a surgical microscope system 100.

Accordingly, the surgical or scientific imaging system or surgical microscope system 100 may comprise an imaging device, such as a microscope 120. In general, a microscope, such as the microscope 120, is an optical instrument that is suitable for examining objects that are too small to be examined by the human eye (alone). For example, a microscope may provide optical magnification of a sample. In the present concept, the optical magnification is (also) provided for at least one optical imaging sensor. The microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often referred to as the "optics carrier" of the imaging system.

There are a variety of different types of surgical or scientific imaging devices. If the imaging device is used in the medical or biological fields, the object being viewed through the imaging device may be a sample of organic tissue, e.g., arranged within a petri dish or present in a part of the body of a patient. In various examples presented here, the imaging device 120 may be a microscope of a surgical microscope system, i.e., a microscope that is to be used during a surgical procedure, such as a neurosurgical procedure (i.e., brain surgery). Accordingly, the sample being viewed through the surgical imaging device may be a sample of organic tissue of a patient and may in particular be the surgical site that the surgeon operates on during the surgical procedure, e.g., the brain. However, the proposed concept is also suitable for other types of surgery, such as eye surgery or cardiac surgery.

Fig. 1b shows a schematic diagram of an example of a surgical or scientific imaging system 100, particularly a surgical microscope system 100 comprising the system 110 and a microscope 120. The surgical microscope system 100 shown in Fig. 1b comprises a number of optional components, such as a base unit 105 (comprising the system 110) with a (rolling) stand, an auxiliary display 140 that may be arranged at the base unit, and a robotic arm that holds the microscope 120 in place and is coupled to both the base unit and the microscope 120. In general, these optional and non-optional components may be coupled to the system 110 which may be configured to control and/or interact with the respective components.

In the proposed concept, the system 110 is used to control various aspects of the surgical or scientific imaging system 100 and/or its imaging device 120. In particular, the system 110 uses a large language model to process natural language input of a user and uses an output of the large language model to control the surgical or scientific imaging system. In other words, the system 110 is configured to obtain a natural language input of a user of the surgical or scientific imaging system, e.g., as voice command (via microphone 130) or as text input (via a user interface of the surgical or scientific imaging system, such as a touchscreen or keyboard).

The system 110 is configured to process the natural language input it obtained from the user using a large language model. A large language model (LLM) is a type of artificial intelligence model designed to understand and generate human language. These models are built using deep learning techniques, particularly neural networks with many layers and parameters. LLMs, such as GPT (Generative Pre-trained Transformer) variants, are trained on vast amounts of text data, enabling them to capture the statistical patterns of language. This training allows them to perform a variety of natural language processing tasks, such as text completion, translation, summarization, and more. The architecture often used for these models is the transformer architecture, which is highly effective at handling the complexities of language due to its ability to dynamically attend to different parts of the input sequence through attention mechanisms.

In literature, the term "LLM" is not well defined. It is usually used to refer to transformer-based language models having with a large number of parameters, such as at least 1 billion parameters. Other sources call language models having less than 5 billion parameters a "small language model". In the context of the present disclosure, the term "LLM" (large language model) is refers to any (transformer-based or diffusion-based) language model having at least 500 million parameters.

In many cases, the input provided to a LLM contains a so-called prompt. A prompt, in the context of a LLM, refers to the initial input that is provided to the model to elicit a response. A prompt can be a question, a partial sentence, or even specific instructions. It guides the model's output by setting the context in which the model should generate a continuation. For example, if a user provides a prompt asking for a summary of a given text, the model will use the information from the prompt to provide a concise summary. In the proposed concept, the prompt may include the natural language input of the user. In addition, the prompt may instruct the LLM to interpret the natural language input of the user, and to generate an output containing at least one parameter for controlling the surgical or scientific imaging system. In addition, the prompt may instruct the LLM to ask the user follow-up questions if the natural language input is ambiguous.

The effectiveness of the model's response often depends on how well the prompt encapsulates the required context and direction for the task at hand. To ensure that the LLM has all the necessary information, the necessary context can be provided to the LLM as part of the prompt. In other words, during runtime, context is provided through the prompt, as the user specifies the initial conditions or instructions to which the model should respond. Providing sufficient context in the prompt helps the model generate more accurate and relevant responses.

Alternatively, the context can be established during training or fine-tuning of the LLM. During training, context is provided to a LLM through the dataset on which it is trained. This involves input-output pairs where the model learns the probabilities of words and phrases following certain contexts in vast corpora of text. Training involves exposing the model to diverse linguistic contexts, allowing it to understand and generate language that is contextually relevant across different scenarios.

In many cases, such as the present case, not only textual data (such as the natural language input of the user) is provided as input (i.e., as part of the prompt) to the LLM. In particular, the natural language input of the user may be provided to the LLM as an audio recording (e.g., instead of the system transcribing the audio recording), or image data of the imaging device 120 may be provided as additional context to the LLM. LLMs that are able to process input other than text are called multimodal LLMs. Multimodal LLMs are advanced machine learning models that can process and generate data across multiple modalities, such as text, images, and audio. These models extend the capabilities of traditional language models by integrating different types of data, allowing for a richer understanding and output. For instance, a multimodal model might be able to generate descriptive text from an image or recognize objects and text within a photo. This integration is achieved by combining different neural network architectures, such as transformers for text and convolutional neural networks (CNNs) for images, enabling the model to process and relate information from various sources simultaneously. These models work by aligning and fusing the representations from different modalities to create a coherent understanding of the input data. The training process involves datasets that pair inputs from multiple types, such as text with corresponding images, allowing the model to learn a shared representation that captures the relationships among the modalities. During inference, the model uses this integrated knowledge to produce cross-modal outputs, such as generating a textual description based on visual content or answering questions about an image.

In some examples, the LLM being used by the system 110 may be hosted by the system 110 itself, i.e., the system 110 may be configured to perform inference on the LLM. Alternatively, the LLM may be hosted by a remote server. In this case, the system 110 may be configured to process the natural language input using the LLM by querying the remote server.

In the proposed concept, the LLM is trained to output, based on the natural language input of the user, at least one parameter for controlling an operation of the surgical or scientific imaging system. Regardless of the specific application to imaging device-specific tasks, this training includes two components. For example, the LLM is trained to follow instructions so that it consistently provides a suitable output based on the natural language input. Thus, the LLM is trained for instruction following. Furthermore, it is trained to provide its output in a suitable format that the system 110 can use to control the surgical or scientific imaging system 100, as the system 110 is configured to control the surgical or scientific imaging system based on the output of the LLM. LLMs trained to provide their output in a consistent, machine-interpretable format (e.g., for input into another machine) are trained to provide structured output. There are numerous LLMs, such as LLMs from the Llama, Phi, Qwen, Mistral or GPT families of LLMs, that are both trained for instruction following and providing structured output and can be used for the proposed task.

Optionally, the LLM may be trained (e.g., fine-tuned), based on a suitable LLM already trained for instruction following and providing structured output, for the specific task at hand: providing the at least one parameter based on the natural language input. This training task is optional, but useful for improving the performance of the LLM. For example, training may be performed starting from an LLM that has been trained for instruction following and providing structured output. Then, a suitable training regimen may be used to fine-tune the LLM for the task at hand. LLMs can be fine-tuned on specific tasks by training them on task-specific datasets using supervised learning techniques. This process involves adjusting the pre-trained model's weights to make it more effective at the specific task. Common algorithms and training methodologies used in this process include supervised fine-tuning with cross-entropy loss for classification tasks, as well as methods like reinforcement learning or proximal policy optimization when optimizing for more complex objectives. For example, in supervised learning, a set of training data may comprise, for each training sample, on the one hand, the natural language input (e.g., along with additional context such as image data) and a corresponding desired at least one parameter to be output by the LLM. When using reinforcement learning, a set of training data may comprise a plurality of training samples (comprising a natural language input, and optionally additional context). In addition, a reward function may be provided to rate the quality of the output provided by the LLM, or the output may be reviewed by a human operator supervising the training.

When starting from a suitable LLM, some techniques may be used to facilitate the fine-tuning. For example, LoRA (Low-Rank Adaptation) is a technique designed to efficiently fine-tune LLMs. It works by introducing trainable low-rank matrices into each layer of the model, allowing for task-specific adaptation without altering the entire model's parameters. This significantly reduces the number of parameters that need updating during fine-tuning, saving computational resources. QLoRA extends this concept by integrating quantization-aware training into the process. By quantizing model weights and activations during training, QLoRA maintains high accuracy while further optimizing memory and speed, allowing for more manageable deployment of large models on limited hardware resources.

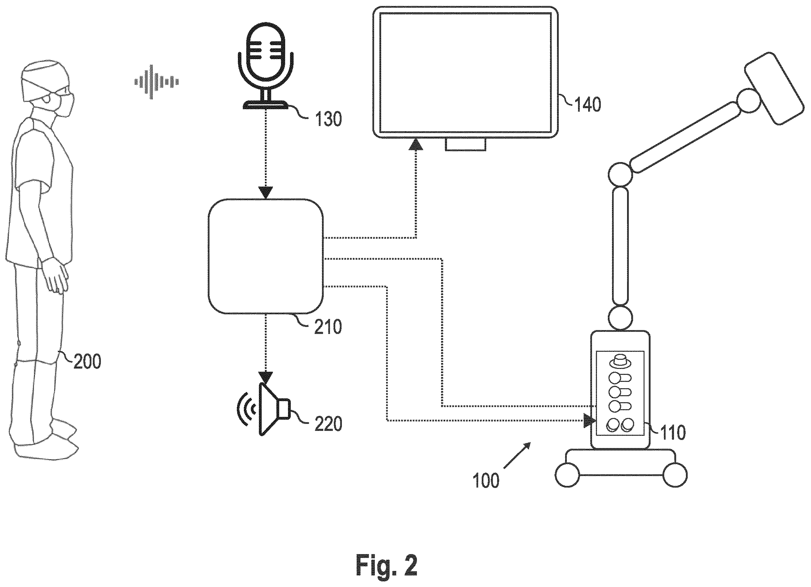

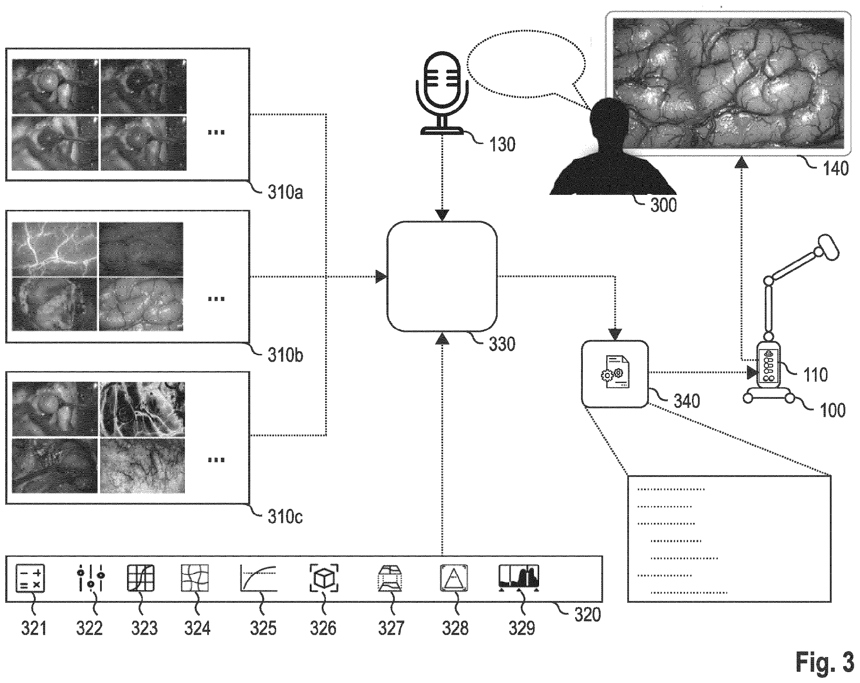

In the present disclosure, the LLM is trained to output at least one parameter for controlling the surgical or scientific imaging system. This may relate to direct control of various mechanical or electrical components of the surgical or scientific imaging system, such as its robotic arm, its zoom or focus functionality, or its illumination functionality. In other words, the system may be configured to control a position of an imaging device of the surgical or scientific imaging system relative to a sample being observed using the imaging device based on the at least one parameter, to control at least one of a zoom functionality or a focus functionality of an imaging device of the surgical or scientific imaging system based on the at least one parameter, and/or to control an illumination system of the surgical or scientific imaging system. These kinds of parameters will be discussed in connection with a first set of examples discussed in connection with Fig. 2. Additionally, or alternatively, in a second set of examples discussed in connection with Fig. 3, the output of the LLM is used to control functionalities of the surgical or scientific imaging device related to image acquisition, image processing and/or provision of a digital view (i.e., display of the processed images). Accordingly, the at least one parameter may include a setting related to at least one of image acquisition, image processing, or display. It is noted that the examples of the first set of examples can be combined with the examples of the second set of examples (and vice versa).

The first set of examples relates to a surgical or scientific microscope (or other imaging device) with a natural language interface using LLMs (LLMs). It provides a surgical microscope system enhanced by an LLM-based interface. This interface enables surgeons to control the microscope using natural language commands. The system interprets spoken instructions (i.e., the natural language input), processes them (using the LLM) while considering contextual information and user preferences, and executes precise control commands (i.e., the at least one parameter). It may provide real-time feedback and dynamic adjustments, thereby improving efficiency, precision, and usability in surgical procedures.

In other systems that do not incorporate such an LLM-based control of parameters of the surgical microscope, manual adjustments are time-consuming and can disrupt the surgical workflow. Additionally, other Al applications may be limited to basic functionalities like autofocus and image recognition, lacking advanced interaction capabilities. Other systems may also lack real-time adaptability and contextual understanding.

A core idea of the first set of examples is to integrate an LLM between the user and the microscope controls. This integration allows surgeons to interact with the microscope and its associated devices, such as sensors, cameras, and microphones, using natural language commands. The LLM interprets these commands by processing contextual information and user preferences, then translates them into precise control instructions. This system may provide dynamic, real-time adjustments that significantly enhance surgical precision and efficiency. It addresses the desire for an intuitive, efficient, and precise control system for surgical microscopes. Additionally, it reduces the time and effort required for manual adjustments and avoids potential errors during surgical procedures.

In the first set of examples, the surgical microscope system 100 is equipped with motorized controls for focus, zoom, and positioning. The Al-powered LLM module has been trained to understand and process natural language commands. The user interface may include a microphone for voice input and an optional screen for visual feedback. Lastly, the control interface (e.g., the system 110) integrates with the microscope and additional devices like sensors, cameras, and microphones to execute commands.

An overview of the surgical microscope system is shown in Fig. 2. Fig. 2 shows an example of LLM-based control of a surgical imaging system. The surgeon 200 issues a natural language command that is picked up by microphone 130. The microphone 130 listens to the spoken natural language command and provides it as input for LLM 200. In other words, voice commands of the surgeon 200 are issued into the microphone 130. These commands are processed by the LLM (LLM), which interprets them and communicates with the microscope for necessary setting adjustments. Additionally, the LLM 210 reads data from the microscope and other sensors (via system 110) to improve control and ensure precise adjustments. The LLM 210 provides an output to control the microscope system 100 through system 110. The LLM 210, through system 110, provides audio feedback to the surgeon and displays video feedback on a monitor. Audio feedback may be provided to the surgeon via an audio output device (e.g., speaker) 220. Video feedback may be provided via display device 140.

Natural language interaction allows the surgeon to issue commands such as "center the field of view on the artery at the bottom-left corner ", or "adjust the observation angle." The LLM processes these commands by considering context, user preferences, and current settings.

In some cases, such as "center the field of view on the artery at the bottom-left corner", the LLM requires additional context, as it needs to determine the position of the artery at the bottom-left corner relative to the current position and orientation of the imaging device. In the proposed concept (in both sets of examples), additional inputs are incorporated from multiple sources such as cameras, eye trackers, and/or buttons. In particular, the digital images recorded by the imaging device 120 of the surgical microscope system 100, or information derived from them, may be provided to the LLM as additional input, to enable the LLM to interpret the natural language input. Thus, the system 110 may be configured to obtain image data of an imaging device 120 of the surgical or scientific imaging system. The system 110 may be configured to provide the image data or data derived from the image data as further input to the LLM. The LLM is trained to use the image data or data derived from the image data as context for evaluating the natural language input. In other words, the LLM may be trained to output, based on the natural language input of the user, and based on the image data or data derived from the image data, the at least one parameter for controlling an operation of the surgical or scientific imaging system.

For example, in the surgical context discussed in connection with Figs. 1b to 3, the image data may be image data of a surgical site. Images of surgical sites often show a) the surgical site itself, i.e., the anatomical features that are exposed at the surgical site, and b) surgical tools being used to operate on the surgical site. In addition, in some cases, the user (surgeon) may use a surgical tool, a finger, or a fiducial pointer to point at something, such as an anatomical feature. To enable the LLM to better understand the context of the surgery, information on the anatomical features (e.g., their position and categorization), information on the surgical tools (e.g., their position and categorization), and information on the pointer may be extracted from the image data and used as context for the LLM. For example, the system 110 may be configured to pre-process the image data to determine at least one of one or more anatomical features, one or more surgical tools or one or more pointers shown in the image data, e.g., using a machine-learning model trained for object detection or image segmentation. The system 110 may provide information on at least one of the one or more anatomical features, the one or more surgical tools or the one or more pointers as the data derived from the image data as further input to the LLM. Alternatively, the LLM may process the image data to detect the anatomical features, surgical tools, or pointers. In other words, the LLM may be trained to determine at least one of one or more anatomical features, one or more surgical tools or one or more pointers shown in the image data, and to provide the at least one parameter for controlling the operation of the surgical or scientific imaging system based on at least one of the one or more anatomical features, the one or more surgical tools or the one or more pointers.

Another additional input that may be used relates to the gaze of the user, which can be determined using an eye tracking system. If there is ambiguity about which anatomical element the user refers to, the system 110 may be configured to highlight options for them. The surgeon can look at the correct element and say "This!" to help the LLM understand their choice. Accordingly, the system may be configured to obtain information on the gaze of the user of the surgical or scientific imaging system, and to provide information on the gaze as further input to the LLM. Other sophisticated inputs may also be used, such as gesture recognition, contactless control, and pointing with hands, fingers, eyes, face, or surgical tools (e.g., using scissors). For example, the system 110 or the LLM may be configured to process the image data or further image data (of a camera observing the user) to obtain such inputs, and to provide data on the respective input as data derived from image data as input to the machine learning model.

In some examples, users may have the ability to assign names to locations or anatomical structures, and to implement workflows. For example, a workflow may include a sequence of operations, such as "zoom out, capture images with all imaging modes, repeat with three observation angles, and then return to the current position." The LLM may be trained to assign the names to the respective location or anatomical structure, and to provide the at least one parameter based on the name(s) assigned to locations or anatomical structures. Additionally, the LLM may be trained to memorize workflows and to output the at least one parameter in accordance with a workflow.

In some examples, the LLM may access multiple data sources such as heartbeat and breathing rates, arterial blood saturation, other imaging modes, and preoperative data, even when the surgeon is not actively accessing them. This may be implemented by a so-called function calling functionality. Function calling refers to the ability of the LLM to invoke predefined functions in order to perform specific tasks or retrieve external information. This capability allows the LLM to extend its functionalities beyond text generation by accessing additional resources or performing computations. By calling a function, the LLM can be provided with supplementary input or data that it can use to generate more accurate or relevant responses. In particular, the LLM may be trained to retrieve at least one of image data, data derived from the image data or information on a gaze of a user of the surgical or scientific imaging system using a function calling functionality.

In some examples, the LLM may be used to monitor specific events. For example, surgeons can instruct the LLM to monitor specific events, such as "inform me when 5-ALA fluorescence is detected" or "alert me if the heart rate goes outside the normal range." Each time the LLM receives a new input (including an image or sensor data being monitored), it may process the input to determine if the event has occurred. If so, it can include a notification in its output. In other words, the system 110 may be configured to provide (e.g., periodically) at least one of information on the state of the surgical or scientific imaging system, image data of an imaging device of the surgical or scientific imaging system, or data derived from the image data of the imaging device of the surgical or scientific imaging system as input for the LLM. For example, the information on the state of the surgical or scientific imaging system may comprise at least one of information on a position and orientation of the imaging device 120 (e.g., relative to a patient), information on a working distance, information on a focus, information on a zoom level, information on an illumination being used, information on imaging modality(s) being used, or the image data. The LLM may be trained to determine one or more events to be detected based on the natural language input of the user, detect the one or more events in the information on the state of the surgical or scientific imaging system, the image data or the data derived from the image data, and provide an output indicating that the one or more events have occurred.

The output of the LLM is used to adjust the imaging device 120 and the imaging system 100. Straightforward commands like "activate autofocus" may result in direct instructions from the LLM. For complex commands, iterative processing may be used: anatomical features may be identified using image recognition, new positions or angles may be calculated, and settings may be adjusted dynamically with feedback provided to the user. For example, the LLM may be trained to perform internal reasoning or iterative task evaluation to perform the operations one after another.

In some cases, additional adjustments may be necessary if the at least one parameter provided by the LLM does not immediately have the desired effect. For example, in a Feedback and Adaptation mechanism, the system 110 may be configured to obtain images or video clips that are captured during adjustments. It may analyze feedback to ensure suitable or optimal settings and may request additional inputs (e.g., capturing images from different angles) to refine these adjustments. In more general terms, the system 110 may be configured to provide, after controlling the surgical or scientific imaging system based on the output of the LLM, information on a state of the surgical or scientific imaging system as input for the LLM. The LLM may be trained to output an update to the at least one parameter for controlling the operation of the surgical or scientific imaging system based on the information on the state.

In some cases, the system 110 may ask the user for confirmation prior to controlling the surgical or scientific imaging systems. For example, the system 110 may be configured to offer (multiple) options for final confirmation. Furthermore, the system 110 may be configured to adapt to user preferences over time, thus improving efficiency and customization.

In some examples, the at least one parameter may be used to control devices that interact with the surgical or scientific imaging system, providing integration with additional devices. For example, the output of the LLM can be used to control various sensors, cameras, and microphones, providing a comprehensive view and better situational awareness. This integration enables multi-modal interaction and feedback, enhancing the system's adaptability and precision. It extends the capabilities of the proposed concept beyond basic microscope control and provides a more holistic approach to surgical assistance by leveraging data from multiple sources for improved decision-making.

The proposed concept according to the first set of examples presents several advantages over other approaches. It reduces manual adjustment time and effort, enhances positioning accuracy and settings precision, provides a user-friendly voice-activated interface, adapts to user preferences for better customization, and integrates additional devices for comprehensive surgical assistance.

More details and aspects of the first set of examples are mentioned in connection with the proposed concept, or one or more examples described above or below (e.g., Fig. 1a to 1b, 3 to 5). The first set of examples may comprise one or more additional optional features corresponding to one or more aspects of the proposed concept, or one or more examples described above or below.

The second set of examples relates to the acquisition, processing and display of image data obtained by an optical imaging sensor of the imaging device 120. In particular, the LLM is used to create or refine imaging modes. It acts as an Al interpreter of natural speech into the algorithmic creation of a microscope imaging mode.

Other microscopes and medical imaging devices usually feature fixed, predefined imaging modes with limited adjustability within each mode. These standard modes present several disadvantages. Firstly, they offer limited flexibility, restricting surgeons to available options that may not be suitable for all surgical situations or personal preferences. Secondly, there is a lack of customization; these devices do not allow the combination of multiple imaging data streams in novel ways or enable adjustments beyond basic settings. Lastly, this setup results in inadequate adaptation, forcing surgeons to modify their techniques according to device capabilities rather than customizing imaging to fit their specific needs.

Creating new imaging modes that take advantage of the diverse data a microscope (or other imaging device) can capture, necessitates significant time and resources from Research & Development (R&D) teams to reprogram the device's code. This process is not only time-consuming and expensive but also often fails to meet all surgeons' individual needs and preferences. Moreover, it cannot be performed or completed during surgical procedures and may require considerable time before deployment. Additionally, it involves substantial resources and costs, making it impractical for creating custom modes tailored to individual surgeons. Furthermore, this method lacks flexibility in response, as it cannot address immediate needs that arise during surgery, thus limiting adaptability.

In many cases, there is limited adjustability within modes, which refers to the minor adjustments that some modes offer, which are insufficient for creating entirely new imaging experiences. This approach may provide insufficient control as users cannot fully customize imaging parameters or dynamically combine different imaging modalities. Moreover, it supports a static workflow and might not allow for on-the-fly adjustments needed in complex or unexpected surgical scenarios.

Overall, other approaches fall short in providing a flexible and efficient method for surgeons to create and customize imaging modes on demand. This limitation hinders their ability to improve imaging that is tailored to specific surgical needs and preferences.

Consequently, there may be a desire for a mechanism that enables surgeons to create custom imaging modes quickly and intuitively, without requiring extensive R&D involvement, thus allowing for real-time adaptation to specific surgical requirements.

The proposed concept discussed in connection with the second set of examples introduces an Al-powered system utilizing a LLM (LLM) to interpret natural language commands from the surgeon and generate code-like instructions to create custom microscope imaging modes in real-time (as at least one parameter for controlling the surgical or scientific imaging system). By leveraging the plurality of imaging data available from the microscope (e.g., white light, various fluorescence channels) and a library of image processing algorithms, the system allows for rapid creation of new imaging modes without requiring time-consuming reprogramming by R&D teams. This empowers surgeons to tailor imaging modes to their specific needs and preferences on-the-fly, enhancing flexibility, improving surgical workflows, and potentially leading to better patient outcomes.

The proposed concept offers a mechanism where surgeons can create custom imaging modes in real-time using natural language commands, without extensive reprogramming. By employing an LLM (LLM) to interpret these commands and generate executable instructions, the system addresses the disparity between the static capabilities of current devices and the dynamic needs of surgeons.

Fig. 3 shows an example of Al (Artificial Intelligence)-driven custom microscope imaging mode creation using natural language commands. An input device, such as a microphone 130, captures the surgeon's 300 voice commands during the procedure. The LLM 330 processes this natural language input to understand and interpret the surgeon's requests in real-time. The LLM has knowledge of (e.g., and optionally can process) the available image feeds, such as raw images 310a, images 310b created using standard microscope imaging modes, or images 310c created using advanced imaging modes. For effective operation, the LLM has access to read current microscope settings like working distance, magnification, illumination mode, and diaphragm settings. It can also control and adjust these settings as necessary to fulfill specific requests, e.g., as discussed in connection with the first set of examples and Fig. 2. An algorithm library 320 is available for the LLM, offering image processing algorithms such as mathematical operations 321, color settings 322, gamma adjustment 323, morphological operations 324, thresholding 325, object detection 326, segmentation 327, sharpness adjustment 328, and histogram operations 329. The system translates voice commands into code-like directives or instructions 340 (imaging mode instructions), e.g., according to a microscope-proprietary file, which are then used to apply image processing algorithms such as color mapping, object detection, and segmentation. Thus, the LLM generates code-like instructions detailing how to process and combine imaging data based on the surgeon's request, which are then executed by the microscope's processing unit 110. Finally, these instructions are transferred to the processing unit via a file or direct digital connection for immediate execution. These customized imaging modes appear on the microscope monitor 140, allowing surgeons to adjust the visualization based on their preferences.

Imaging modes are based on so-called image processing pipelines. In an image processing pipeline, one or more source images (i.e., types of image data) are processed using a sequence of one or more image processing operations, with one (or two, in stereoscopic imaging) image(s) being output by the image processing pipeline that represent(s) the image processing operation(s) being performed on the respective source. In the context of the present disclosure, the LLM may be used to generate a new image processing pipeline and/or modify an existing image processing pipeline based on the natural language input provided by the user. In other words, the LLM may be trained to output information on a configuration of an image processing pipeline based on the natural language input of the user. In particular, the user may describe their desired visualization, and the LLM outputs a configuration of an image processing pipeline that yields the desired visualization. In other words, the LLM may be trained to output the information on the configuration of the image processing pipeline based on natural language describing a desired visual output of the image processing pipeline. The system 110 then uses that image processing pipeline to process image data and provide a display signal for a display device 140 of the surgical imaging system. In particular, the system 110 may be configured to process image data or pre-processed image data of the imaging device 120 of the surgical or scientific imaging system using an image processing pipeline that is based on the information on the configuration of the image processing pipeline output by the LLM.

As shown in Fig. 3, two main components are defined in an image processing pipeline - the source image(s), and the image processing operation(s) being used to process the source image(s). Thus, the image processing pipeline may be processed image data or pre-processed image data of the imaging device of the surgical or scientific imaging system. One sub-task in outputting the configuration of an image processing pipeline thus includes selecting one or more appropriate source images. In other words, the LLM may be trained to select one or more instances of a plurality of available types of image data or pre-processed image data based on the natural language input of the user. In this context, the plurality of available types of image data refers to any type of image data (e.g., raw images 310a, images of standard microscope imaging modes 310b, images of advanced imaging modes 310c) that are available for processing at the system 110. This may include raw, unprocessed images and pre-processed images (corresponding to standard or advanced imaging modes). Additionally, or alternatively (in case there is only one source image available), the LLM may select image processing operations to be applied on the source image(s). In other words, the image processing pipeline may comprise a sequence of (one or more) image processing operations. The LLM may be trained to select one or more image processing operations for the image processing pipeline from a library 320 of available image processing operations based on the natural language input of the user. For example, as shown in Fig. 2, the library of available image processing operations 320 (e.g., algorithmic tools) may comprise at least one of one or more mathematical operations 321, one or more morphological operations 324, a gamma adjustment operation 323, one or more color setting operations 322, one or more thresholding operations 325, a sharpness adjustment operation 328, one or more histogram-based operations 329, an image segmentation operation 327, or an object detection operation 326.

To improve the performance of the LLM when selecting types of image data and image processing operations, context may be provided that allows the LLM to understand which features are best shown in which type of image data, and which image operations have what effect. This context may be provided at runtime. In this case, the system 110 may be configured to provide a data structure comprising information on available image processing operations and/or information on available types of image data or pre-processed image data as input for the LLM. Alternatively, or additionally, this context may be provided during training of the LLM. In other words, the LLM may be trained based on the data structure comprising information on available image processing operations and/or information on available types of image data or pre-processed image data.

To ensure that the output of the image processing pipeline is at least somewhat usable, a limited set of functional tests may be performed to evaluate the resulting image processing pipeline. In other words, the system may be configured to perform a pre-defined set of functional tests on the image processing pipeline before processing the image data or pre-processed image data of an imaging device of the surgical or scientific imaging system using the image processing pipeline. For example, the pre-defined set of functional tests may include a test based on a histogram of the image being output by the image processing pipeline. If the histogram indicates that the image is too dark or too bright, then the image processing pipeline might not be used. Similarly, if a delay introduced by the image processing pipeline is too large (e.g., larger than 100 ms), then the image processing pipeline might not be used.

In the following, an example process flow is presented. The process begins with a voice command input from the surgeon, such as "Show me the anatomy in black and white, the ICG fluorescence in green, and the 5-ALA in red." Microphones capture this command, which is then converted to text using speech recognition software or by the LLM. Next, the LLM interprets and analyzes the textual command to understand the requested imaging mode, identifying the specific type of imaging data (e.g., white light, ICG fluorescence, 5-ALA fluorescence) to use and desired processing steps (e.g., converting anatomy to black and white, mapping fluorescence to specific colors). The LLM may check available imaging data streams and current microscope settings to determine if any adjustments are needed for acquiring the required data. It then selects appropriate algorithms (image processing operations) from its library to process the imaging data and generates code-like instructions (as at least one parameter for controlling the surgical imaging system) detailing the processing steps, such as converting the white light image to grayscale, applying color mapping to fluorescence images, and overlaying images with transparency adjustments. If necessary, the LLM adjusts microscope settings to optimize image acquisition for the requested mode by switching illumination modes or adjusting the diaphragm. The generated instructions may be sent to the microscope's processing unit (i.e., system 110), which may execute them in real-time. Finally, the processed, custom imaging mode may be displayed to the surgeon (e.g., immediately), allowing the procedure to continue seamlessly without interruption.

To illustrate how this system functions in practice, the following example use cases may be considered. In Case 1, a surgeon gives the command: "Show me white light with overlapping 400 and 560 nm fluorescence in blue." The LLM interprets this by combining images of white light with fluorescence at 400 nm and 560 nm. It then applies a blue color map to these combined fluorescence images before overlaying them onto the original white light image. In Case 2, a command such as "Enhance the contrast of the blood vessels and display them in high definition" is interpreted by accessing imaging data that emphasizes blood vessels. The system applies contrast enhancement and sharpness adjustments before displaying an image where vessel visibility is significantly improved.

The proposed concept provides a number of features to improve the definition of imaging modes. For example, the proposed concept uses natural language interaction, allowing surgeons to utilize intuitive voice commands without having to master specific syntax. It enables real-time customization to generate and modify imaging modes immediately, reducing any potential disruptions during surgery. The proposed concept eliminates bottlenecks in research and development by reducing the need for specialized teams to create new imaging modes, thereby conserving both time and resources. It provides enhanced flexibility, permitting the integration of complex imaging data and processing algorithms tailored to specific surgical requirements. It also improves workflow efficiency by reducing the necessity for manual adjustments and physical contact with the microscope, which helps maintain sterility and surgical focus. The proposed concept is adaptable to individual surgeon preferences, enabling personalized adjustments in imaging modes (i.e., "personalized imaging modes") that align with their specific techniques.

In some implementations, instead of or in addition to voice-based natural language input, text-based inputs through keyboards, touchscreens, or other devices may be used. This is particularly beneficial in noisy settings or for those preferring non-voice interactions. In some examples, gesture recognition may be used, allowing surgeons to adjust settings through hand movements or touchless interfaces instead of voice commands. Moreover, inclusion of multi-language support by the LLM may enable non-English-speaking surgeons to operate the system efficiently.

The proposed concept can be integrated with larger systems and existing products. The proposed concept is compatible with various advanced imaging modalities, supporting both standard options like white light and fluorescence as well as more sophisticated ones such as GLOW400, GLOW800, laser speckle, and thermal imaging. It also provides compatibility with existing microscopes, as the proposed concept can be integrated seamlessly with existing microscope platforms that possess the necessary processing capabilities, thus extending their functionality without necessitating new hardware. Workflow integration allows it to connect with hospital systems for seamless recording and documentation, ensuring that customized imaging modes become a part of the patient's medical record.

The following text outlines a customer workflow divided into three main stages: Preparation, During Surgery, and Post-Procedure. In the preparation stage, the surgeon ensures that both the microscope and the LLM system are operational. Additionally, microphones and any necessary input devices are set up and tested to ensure everything functions correctly. During surgery, the surgeon issues voice commands to adjust imaging modes as needed. These commands are processed by the system (e.g., system 110 and the LLM) in real-time, with updates displayed immediately. This setup allows the surgeon to concentrate on the procedure without needing manual adjustments. In the post-procedure stage, it is possible to save custom imaging modes and settings that were used during surgery. These can be reviewed or reused for future procedures, ensuring continuity and efficiency in surgical practices.

The proposed concept, according to the second set of examples, allows the creation and customization of custom imaging modes through natural language voice commands. AI or LLM integration is used to interpret complex, custom requests and generate corresponding imaging modes. The Al or LLM can access a library of algorithms for real-time image processing. The proposed concept eliminates the need for R&D involvement in creating new imaging modes. In the proposed concept, natural language commands can be used to adjust imaging modes on-the-fly. It can process complex requests to interpret and execute them accurately without prior programming.

More details and aspects of the second set of examples are mentioned in connection with the proposed concept, or one or more examples described above or below (e.g., Fig. 1a to 2, 4 to 5). The second set of examples may comprise one or more additional optional features corresponding to one or more aspects of the proposed concept, or one or more examples described above or below.

In the proposed surgical or scientific imaging system of Figs. 1a to 3, the optical imaging sensor(s) is/are used to provide the aforementioned image data. Accordingly, the optical imaging sensor(s), which may be part of the imaging device 120 (e.g., of the microscope) may be configured to generate the image data. For example, the optical imaging sensor(s) of the imaging device 120 may comprise or be an APS (Active Pixel Sensor)- or a CCD (Charge-Coupled Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensor by circuitry of the imaging sensor to perform the imaging.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer, or a programmable hardware component operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system 110 and of the surgical or scientific imaging device 100 are mentioned in connection with the proposed concept, or one or more examples described above or below (e.g., Fig. 1a to 5). The system 110 and the surgical or scientific imaging device 100 may comprise one or more additional optional features corresponding to one or more aspects of the proposed concept, or one or more examples described above or below.

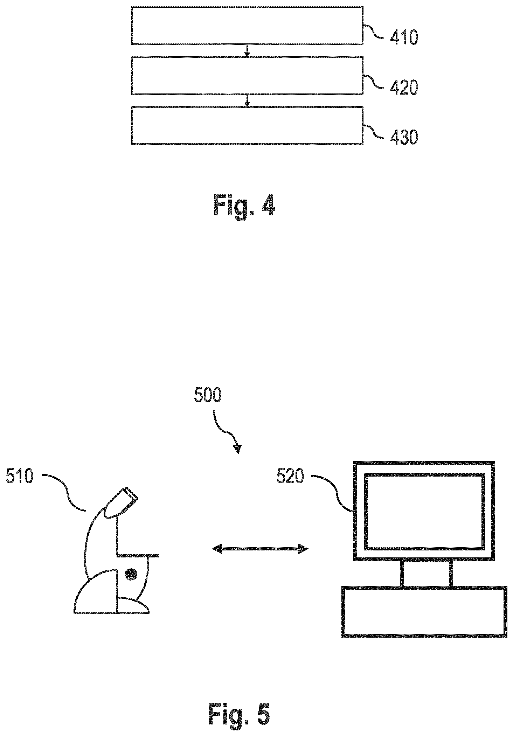

Some aspects of the present disclosure relate to a method for a surgical or scientific imaging system, such as the surgical or scientific imaging system 100 of Figs. 1b to 3. For example, the method may be performed by the surgical or scientific imaging system 100 shown in Figs. 1b, 2 and 3, and in particular by the system 110 shown in Figs. 1a to 3. The method comprises obtaining 410 a natural language input of a user of the surgical or scientific imaging system. The method comprises processing 420 the natural language input using a large language model. The large language model is trained to output, based on the natural language input of the user, at least one parameter for controlling an operation of the surgical or scientific imaging system. The method comprises controlling 430 the surgical or scientific imaging system based on the output of the large language model. Features discussed in connection with the surgical or scientific imaging system of Figs. 1a to 3 may likewise be included in the method of Fig. 4.

More details and aspects of the method are mentioned in connection with the proposed concept, or one or more examples described above or below (e.g., Fig. 1a to 3, 5). The method may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device or imaging system, such as the surgical or scientific imaging device 120 or the surgical or scientific imaging system 100 of Fig. 1a and/or 1b comprising a system 110 as described in connection with one or more of the Figs. 1a to 4. Alternatively, an imaging device, such as a microscope or an exoscope, may be part of or connected to a system as described in connection with one or more of the Figs. 1a to 4. Fig. 5 shows a schematic illustration of a system 500 configured to perform a method described herein. The system 500 comprises an imaging device 510, such as a microscope or exoscope, and a computer system 520. The imaging device 510 is configured to take images and is connected to the computer system 520. The computer system 520 is configured to execute at least a part of a method described herein. The computer system 520 may be configured to execute a machine learning algorithm. The computer system 520 and imaging device 510 may be separate entities but can also be integrated together in one common housing. The computer system 520 may be part of a central processing system of the imaging device 510 and/or the computer system 520 may be part of a subcomponent of the imaging device 510, such as a sensor, an actor, a camera, or an illumination unit, etc., of the imaging device 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer, or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all the functionalities of the methods described herein. In some embodiments, a field programmable gate array may cooperate with a microprocessor to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g., by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the actions taken, a reward is calculated. Reinforcement learning is based on training the one or more software agents to choose the actions such that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train, or use a machine-learning model. The term "machine-learning model" may denote a data structure and/or set of rules that represent the learned knowledge (e.g., based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receive input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e., to achieve a desired output for a given input.

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100:: Surgical or scientific imaging system
- 110:: System
- 112:: Interface
- 114:: Processor
- 116:: Storage device
- 120:: Imaging device
- 130:: Microphone
- 140:: Display device
- 200:: Surgeon
- 210:: LLM
- 220:: Audio output device
- 300:: Surgeon
- 310a:: Raw images
- 310b:: Images created using standard microscope imaging modes
- 310c:: Images created using advanced microscope imaging modes
- 320:: Algorithm library
- 321:: Mathematical operations
- 322:: Color settings
- 323:: Gamma adjustment
- 324:: Morphological operations 324
- 325:: Thresholding
- 326:: Object detection
- 327:: Segmentation
- 328:: Sharpness adjustment
- 329:: Histogram operations
- 330:: LLM
- 340:: Code-like directives or instructions
- 410:: Obtaining a natural language input
- 420:: Processing the natural language input using an LLM
- 430:: Controlling a surgical or scientific imaging system
- 500:: System
- 510:: Imaging device
- 520:: Computer system

## Claims

1. A system (110; 520) for a surgical or scientific imaging system (100; 500), the system comprising one or more processors and one or more storage devices, wherein the system is configured to:
obtain a natural language input of a user of the surgical or scientific imaging system;
process the natural language input using a large language model, wherein the large language model is trained to output, based on the natural language input of the user,
at least one parameter for controlling an operation of the surgical or scientific imaging system; and
control the surgical or scientific imaging system based on the output of the large language model.

2. The system according to claim 1, wherein the system is configured to obtain image data of an imaging device (120; 510) of the surgical or scientific imaging system, and to provide the image data or data derived from the image data as further input to the large language model, with the large language model being trained to output, based on the natural language input of the user and based on the image data or data derived from the image data, the at least one parameter for controlling an operation of the surgical or scientific imaging system.

3. The system according to claim 2, wherein the image data is image data of a surgical site,
wherein the system is configured to pre-process the image data to determine at least one of one or more anatomical features, one or more surgical tools or one or more pointers shown in the image data, and to provide information on at least one of the one or more anatomical features, the one or more surgical tools or the one or more pointers as the data derived from the image data as further input to the large language model,
or wherein the large language model is trained to determine at least one of one or more anatomical features, one or more surgical tools or one or more pointers shown in the image data, and to provide the at least one parameter for controlling the operation of the surgical or scientific imaging system based on at least one of the one or more anatomical features, the one or more surgical tools or the one or more pointers.

4. The system according to one of the claims 1 to 3, wherein the system is configured to obtain information on a gaze of a user of the surgical or scientific imaging system, and to provide information on the gaze as further input to the large language model.

5. The system according to one of the claims 1 to 4, wherein the system is configured to provide, after controlling the surgical or scientific imaging system based on the output of the large language model, information on a state of the surgical or scientific imaging system as input for the large language model, with the large language model being trained to output an update to the at least one parameter for controlling the operation of the surgical or scientific imaging system based on the information on the state.

6. The system according to one of the claims 1 to 5, wherein the system is configured to provide at least one of information on a state of the surgical or scientific imaging system, image data of an imaging device of the surgical or scientific imaging system, or data derived from the image data of the imaging device of the surgical or scientific imaging system as input for the large language model, with the large language model being trained to determine one or more events to be detected based on the natural language input of the user, detect the one or more events in the information on the state of the surgical or scientific imaging system, the image data or the data derived from the image data, and provide an output indicating the one or more events having occurred.

7. The system according to one of the claims 1 to 6, wherein the system is configured to control a position of an imaging device of the surgical or scientific imaging system relative to a sample being observed using the imaging device based on the at least one parameter,
and/or wherein the system is configured to control at least one of a zoom functionality or a focus functionality of an imaging device of the surgical or scientific imaging system based on the at least one parameter,
and/or wherein the at least one parameter includes a setting related to at least one of image acquisition, image processing, or display.

8. The system according to one of the claims 1 to 7, wherein the large language model is trained to output information on a configuration of an image processing pipeline based on the natural language input of the user, with the system being configured to process image data or pre-processed image data of an imaging device of the surgical or scientific imaging system using an image processing pipeline that is based on the information on the configuration of the image processing pipeline output by the large language model.

9. The system according to claim 8, wherein an image processing pipeline comprises a sequence of image processing operations, wherein the large language model is trained to select one or more image processing operations for the image processing pipeline from a library of available image processing operations based on the natural language input of the user.

10. The system according to claim 9, wherein the library of available image processing operations comprises at least one of one or more mathematical operations, one or more morphological operations, a gamma adjustment operation, one or more thresholding operations, a sharpness adjustment operation, one or more histogram-based operations, an image segmentation operation, or an object detection operation.

11. The system according to one of the claims 8 to 10, wherein the image processing pipeline is configured to process image data or pre-processed image data of an imaging device of the surgical or scientific imaging system, with the large language model being trained to select one or more instances of a plurality of available types of image data or pre-processed image data based on the natural language input of the user.

12. The system according to one of the claims 8 to 11, wherein the system is configured to provide a data structure comprising information on available image processing operations and/or information on available types of image data or pre-processed image data as input for the large language model, or wherein the large language model is trained based on the data structure comprising information on available image processing operations and/or information on available types of image data or pre-processed image data.

13. The system according to one of the claims 8 to 12, wherein the large language model is trained to output the information on the configuration of the image processing pipeline based on natural language describing a desired visual output of the image processing pipeline.

14. A method for a surgical or scientific imaging system (100; 500), the method comprising:
obtaining (410) a natural language input of a user of the surgical or scientific imaging system;
processing (420) the natural language input using a large language model, wherein
the large language model is trained to output, based on the natural language input of the user, at least one parameter for controlling an operation of the surgical or scientific imaging system; and
controlling (430) the surgical or scientific imaging system based on the output of the large language model.

15. A computer program having a program code for performing a method according to claim 14 when the program is executed on processor.
